# EUROPEAN PATENT SPECIFICATION

(11) **EP 2 938 394 B1**
(45) Date of publication and mention of the grant of the patent: **13.03.2019**
(21) Application number: 12816623.8
(22) Date of filing: 28.12.2012
(51) Int. Cl.: A61N 1/05, A61B 5/04

(54) **TIP ELEMENTS FOR COCHLEAR IMPLANTS**
SPITZENELEMENTE FÜR COCHLEARIMPLANTATE
ÉLÉMENTS D'EXTRÉMITÉ POUR IMPLANTS COCHLÉAIRES

(43) Date of publication of application: 04.11.2015
(73) Proprietor: Advanced Bionics AG, 8712 Staefa (CH)
(72) Inventor: DOWNING, Mark B., Valencia, CA 91354 (US); KULKARNI, Abhijit, Newbury Park, California 91320 (US); HARTLEY, Lee F., Carlsbad, CA 92009 (US)
(74) Representative: Schwan Schorer & Partner mbB
(86) International application number: PCT/US2012/072138
(87) International publication number: WO 2014/105059

(56) References cited:
- WO-A1-2011/053766
- US-A1- 2005 216 073
- US-A1- 2006 020 318
- US-A1- 2007 225 787
- US-A1- 2011 098 719

## Description

### BACKGROUND

A variety of implantable devices can be used to augment or replace natural biological functions. For example, cochlear implant systems can be used to provide a sense of hearing to deaf or severely hard of hearing patients. Cochlear implant systems typically include an external portion and an implantable portion. The implantable portion includes an electrode array that is inserted into the cochlea. WO 2011/053766 A1 relates to a stylet which is to be inserted into a lumen of the lead of a cochlear implant device for inserting the electrode array of the lead into the cochlea; once the electrode array has been fully inserted into the cochlea, the stylet is withdrawn from the lead. The tip of the stylet is provided with a pressure sensor which may be implemented as a fiber optic sensor, such as a Fabry-Perrot sensor. The pressure sensor serves to monitor the forces acting on the lead during insertion into the cochlea.

US 2011/098719 A1 relates to a cochlear implant system according to the preamble of claim 1.

### SUMMARY OF THE INVENTION

The invention relates to a cochlear implant system as defined in claim 1. Any methods disclosed hereinafter do not form part of the scope of the invention.

### BRIEF DESCRIPTION OF THE DRAWINGS

The accompanying drawings illustrate various examples of the principles described herein and are a part of the specification. The illustrated examples are merely examples and do not limit the scope of the claims.
Fig. 1 is a diagram of a cochlear implant system, including an internal portion that is surgically placed within a patient and an external portion, according to one example of principles described herein.
Fig. 2 is top view of the internal portion of the cochlear implant, according to one example of principles described herein.
Fig. 3 is a cross sectional view of a cochlea with an electrode array being inserted into the cochlea, according to one example of principles described herein.
Figs. 4A-4C show electrode arrays that include various tip elements, according to one example of principles described herein.
Fig. 5A is a side view of an electrode array with a split tip electrode, according to one example of principles described herein.
Fig. 5B is an end view of the electrode array with a split tip electrode showing the electromagnetic field lines produced by the split tip electrodes, according to one example of principles described herein.
Figs. 6A-6D are cross sectional diagrams of various tip element designs, according to one example of principles described herein.
Fig. 7A is a diagram of an illustrative cochlear implant system that uses a tip element to detect cochlear conditions during insertion of an electrode array, according to one example of principles described herein.
Fig. 7B is a diagram of insertion of an electrode array into a cochlea, according to one example of principles described herein.
Figs. 8A and 8B are flowcharts of illustrative methods for using a cochlear implant system with a tip element to detect cochlear conditions during insertion of an electrode array, according to one example of principles described herein.
Throughout the drawings, identical reference numbers designate similar, but not necessarily identical, elements.

### DETAILED DESCRIPTION

The implanted portion of a cochlear prosthesis includes an electrode array that is inserted into the cochlea. The insertion of the electrode array into the cochlea places an array of electrodes proximal to the tonotopically arranged nerves in the modiolus of the cochlea. Monitoring the interaction between the electrode array and cochlea during insertion can provide valuable information about insertion forces and the biological function of the cochlea.

In the following description, for purposes of explanation, numerous specific details are set forth in order to provide a thorough understanding of the present systems and methods. It will be apparent, however, to one skilled in the art that the present apparatus, systems and methods may be practiced without these specific details. Reference in the specification to "an example" or similar language means that a particular feature, structure, or characteristic described in connection with the example is included in at least that one example, but not necessarily in other examples.

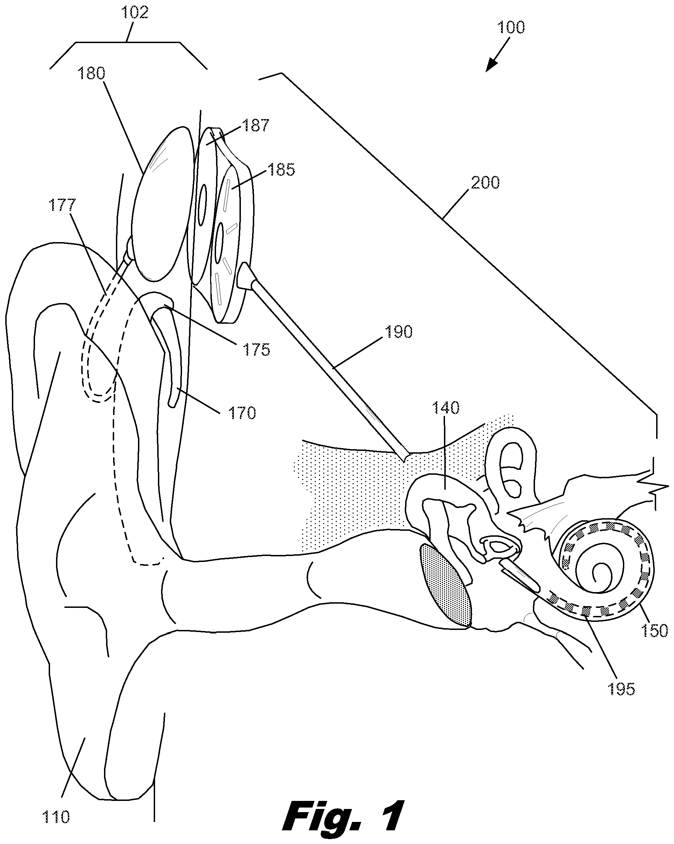

Fig. 1 is a diagram showing one illustrative example of a cochlear implant system (100) that includes an internal implanted portion (200) and an external portion (102). The cochlear implant (100) provides a sense of sound to a person who is profoundly deaf or severely hard of hearing. In many cases, deafness is caused by the absence or destruction of the hair cells in the cochlea, i.e., sensorineural hearing loss. In the absence of properly functioning hair cells, there is no way auditory nerve impulses can be directly generated from ambient sound. Thus, conventional hearing aids, which amplify external sound waves, provide no benefit to persons suffering from complete sensorineural hearing loss.

The external portion (102) of the cochlear implant system (100) can include a Behind-The-Ear (BTE) unit (175), which contains the sound processor and has a microphone (170), a cable (177), and a transmitter (180). The microphone (170) picks up sound from the environment and converts it into electrical impulses. The sound processor within the BTE unit (175) selectively filters and manipulates the electrical impulses and sends the processed electrical signals through the cable (177) to the transmitter (180). The transmitter (180) receives the processed electrical signals from the processor (175) and transmits them to the implanted antenna assembly (187) by electromagnetic transmission.

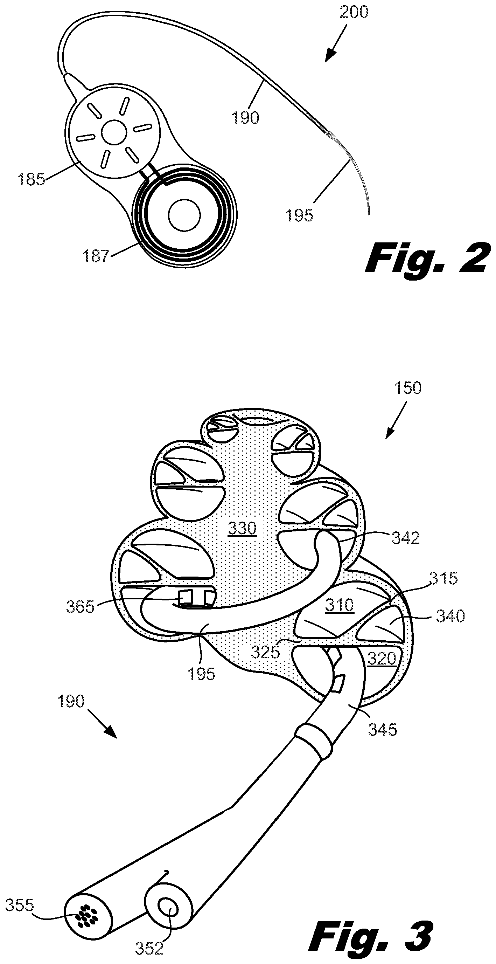

The internal implanted portion (200) of the cochlear implant includes an electrode array (195) that is surgically placed within the patient's cochlea. Unlike hearing aids, the cochlear implant (100) does not amplify sound, but works by directly stimulating any functioning auditory nerve cells inside the cochlea (150) with electrical impulses representing the ambient acoustic sound. This bypasses the defective cochlear hair cells that normally transduce acoustic energy into electrical energy. The implanted portion (200) of the cochlear implant system is shown in Fig. 1 in its implanted configuration and in Fig. 2 before implantation. The internal portion (200) of the cochlear implant (100) includes an internal processor (185), an antenna assembly (187), and a cochlear lead (190) having an electrode array (195). The internal processor (185) and antenna assembly (187) are secured beneath the user's skin, typically above and behind the external ear (110). The antenna assembly (187) receives signals and power from the transmitter (180). The internal processor (185) receives these signals and operates on the signals to generate modified signals. These modified signals are then sent through the cochlear lead (190) to the electrode array (195), which is at the distal portion of the cochlear lead (190) and is implanted within the cochlea (150). The electrode array uses the modified signals to provide electrical stimulation to the auditory nerve (160).

Fig. 3 is a cross sectional view of a cochlea (150) and shows an illustrative electrode array (195) placed within the cochlea (150). The structure of the cochlea (150) is a hollow, helically coiled, tubular bone, similar to a nautilus shell. The coiled tube is divided through most of its length into three fluid-filled spaces (scalae). The scala vestibuli (310) is partitioned from the scala media (340) by Reissner's membrane (315) and lies superior to it. The scala media (340) in a functioning ear contains structures (hair cells) and nerve endings (the organ of Corti) that transduce sound waves passing through the scala into nerve impulses. The scala tympani (320) is partitioned from the scala media (340) by the basilar membrane (325) and lies inferior to it.

A typical human cochlea includes approximately two and a half helical turns of its various constituent channels around the modiolus (330). The modiolus (330) is a conical shaped central axis in the cochlea. The modiolus is formed from spongy bone and has the spiral ganglion located inside of it. The spiral ganglion is a group of nerve cells that detect and transmit a representation of sound from the cochlea to the brain. In a properly functioning cochlea, the dendrites of the spiral ganglion make synaptic contact with the base of hair cells in the organ of Corti. The dendrites are connected to axons of the spiral ganglion. The axons are bundled together to form the auditory portion of the eighth cranial nerve. The hair cells trigger action potentials in the dendrites in response to sound waves passing through the cochlea. These action potentials pass through the spiral ganglion to the brain. To compensate for absent or malfunctioning hair cells, the cochlear lead (190) directly stimulates the spiral ganglion in the modiolus. This bypasses the defective hair cells and/or nerve endings in the organ of Corti.

The cochlear lead (190) includes a flexible body (345) and an electrode array (195) at the distal end of the flexible body. A number of medial electrodes (365) are arranged along one side of the flexible body (345). This array of medial electrodes terminates near the tip (342) of the flexible body (345). Conductors (355) pass through the flexible body to connect to each of electrodes (365). The flexible body may include a number of additional features, such a lumen (352). A stylet can be inserted into the lumen (352) to stabilize and control the electrode array during insertion.

The electrode array (195) is inserted into one of the scalae, typically the scala tympani (320), to bring the individual medial electrodes (365) into close proximity with the tonotopically organized spiral ganglion nerves in the modiolus. Specifically, the electrode array (195) coils around the modiolus of the cochlea with the side of the electrode array that contains the array of medial electrodes on the same side as the modiolus. By placing the array of medial electrodes in proximity with the spiral ganglion, voltages applied by the electrodes can selectively trigger the generation of electrical impulses within the spiral ganglion.

Current electrode leads are not designed to monitor and evaluate the electrophysiologic parameters of the cochlea during electrode insertion. The monitoring of electrophysiologic parameters of during insertion can be useful in preserving residual hearing. Additionally, it may be useful as a tool during automated insertions, particularly for "guided" electrodes.

Tip elements are not found on any conventional cochlear electrode array. As discussed above, the medial electrodes on conventional electrode arrays are designed to be placed to stimulate the ganglion cells that are adjacent in the modiolar direction to the electrode array. A tip element offers the potential to measure normal auditory potentials during insertion and provides for novel stimulation paradigms. Specifically, the tip elements may be used to stimulate firing of the dendrites connected to the base of hair cells. This can provide a measurement of extent of damage to the hair cells. Consequently, the tip element may direct its stimulation to a different location within the cochlea than the linear array of medial electrodes along one side of the flexible body. Further, the tip element may be used to evaluate the function of portions of the cochlea that the electrode array has not yet passed through. This can be performed by directing stimulation out of the tip of the electrode toward areas in front of the electrode array. Further, the stimulation maybe primarily directed in directions that do not intersect with the modiolus. A variety of tip element designs are described below.

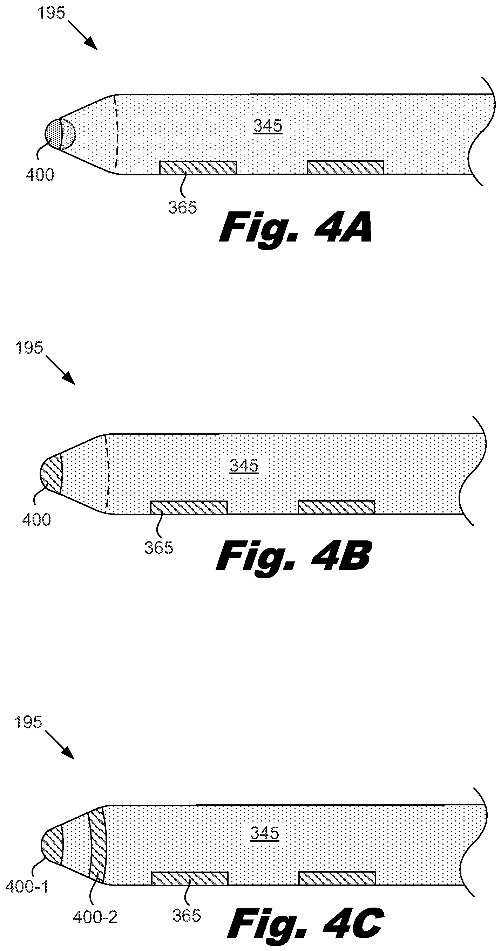

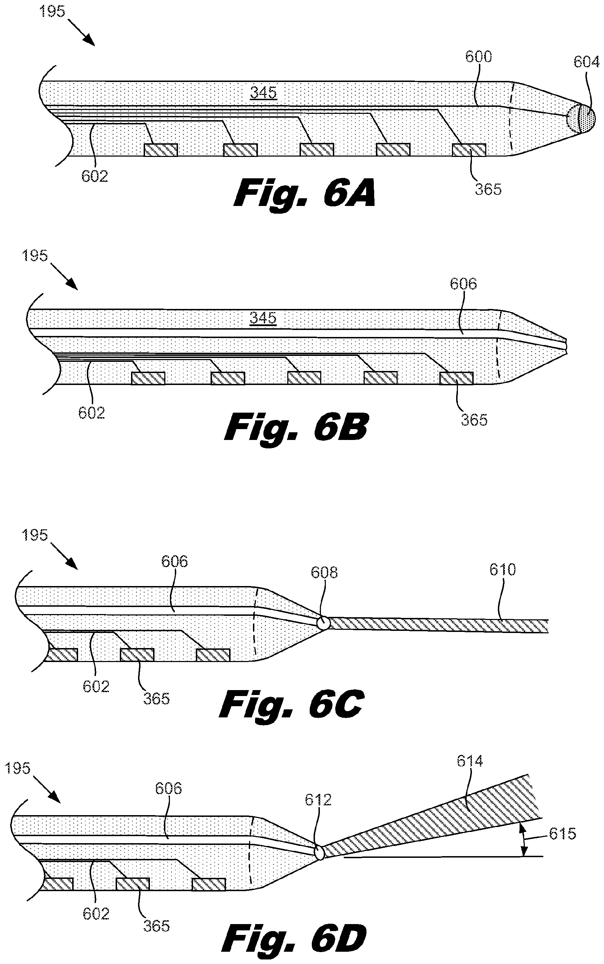

Figs. 4A-4C show distal portions of cochlear implants showing a number of tip element configurations. In these examples, the tip element is the most prominent distal part of the electrode array. In Fig. 4A, the electrode array (195) includes a flexible body (345), with an array of medial electrodes (365) along one side of the flexible body. In this example, the tip element is a ball electrode (400) placed at the distal tip of the electrode array (195). A description of a method for forming a ball tip element is given below with respect to Fig. 6A. Fig. 4B shows an electrode array (195) with a tip element (400) that has a conformal cone shape.

Fig. 4C shows an electrode array (195) that includes a tip element with a split electrode (400-1, 400-2). The split electrode allows for differential measurements and/or stimulation. In some examples, one electrode in the tip element may be used to stimulate surrounding tissue and the other electrode may be used to sense the response. In this example, the split electrode includes a central electrode (400-1) at the distal tip of the electrode array (195) and a ring electrode (400-2) around the diameter of the flexible body (345). The ring electrode (400-2) and the central electrode (400-1) are laterally separated by a portion of the flexible body. This allows the ring and central electrodes (400) to be controlled independently.

The return path for electricity produced by the various tip electrodes may be an implant ground or an electrode in the linear array. Additionally or alternatively, a split electrode may operate as a differential pair, with a first tip electrode being electrified and a second tip electrode acting as an electrical ground.

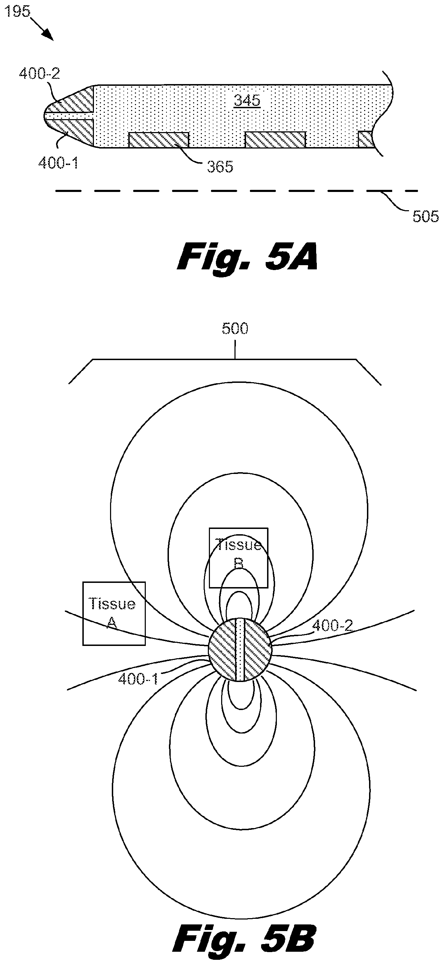

Fig. 5A shows an electrode array (195) with a split tip electrode (400-1, 400-2) and a linear array of electrodes (365) adjacent to the medial wall (505) of the cochlea. The split tip electrode (400) generates a non-uniform distribution of the electromagnetic field around the tip. This can be useful for a variety of reasons, including selective sensing and/or stimulation of surrounding tissue. For example, the split electrode may be specifically designed to stimulate the organ of Corti and auditory elements it contains. In one example, the split electrodes can be separately activated depending on which electrode is closer in proximity to the auditory elements that generate auditory potentials. For example, if a right ear is being implanted, a first split tip electrode may be used to measure the auditory potentials. If a left ear is being implanted, a second split tip electrode can be used.

Fig. 5B is an end view of the electrode array with the split tip (400) showing the non-uniform electromagnetic field (500). Tissue B is subject to an electrical field that is significantly stronger than the electrical field experienced by tissue A, which is at a different radial location but at a similar distance from the electrode array. For example, tissue B may be the organ of Corti and tissue A may be the modiolus. Thus, the orientation and construction of the tip element is designed to trigger nerve impulses in the organ of Corti to test the function of the cochlea's natural sound transducing mechanism rather than to stimulate nerve impulses that originate in the modiolus.

Fig. 6A is a side cross-sectional view of an electrode array (195) with a linear array of electrodes (365) and connecting wires (602) encapsulated in a flexible body (345). The tip electrode (604) is a ball electrode that extends out of the distal end of the flexible body (345). This configuration may have a number of benefits, including being easily formed. For example, a ball electrode (604) may be formed from the wire (600). The wire may be formed from any of a variety of biologically compatible metals, including platinum and platinum alloys. To form the ball electrode (604), a flame is applied to the end of the platinum wire (600). The flame melts the wire and the surface tension of the molten metal forms a sphere that hangs from the tip of the unmelted portion of the wire. The flame continues to melt the wire and the size of the sphere of melted metal increases. When the ball reaches the desired size, the heat is removed and the molten ball of metal solidifies to form the ball electrode (604). The ball electrode and connected wire can then be incorporated into the electrode array during the molding process.

The tip electrode(s) can be formed in a variety of ways, including the electrode formation processes described in U.S. Pat. No. 4,686,765 to Byers et al. and U.S. Pat. No. 4,819,647 to Byers et al., which are incorporated by reference herein in their entireties. For example, U.S. Pat. No. 4,819,647 in column 5, line 40 through column 7, line 5 describes an illustrative technique for forming an electrode by forming a ball electrode, swaging the ball electrode into a desired shape, and optionally coating the electrode.

Fig. 6B is a cross sectional diagram of a distal portion of an electrode array (195) that includes an optical fiber (606) passing through the flexible body (345) and terminating at the tip of the electrode array. The optical fiber (606) is capable of delivering therapeutic optical energy to tissue in front of the electrode array. Additionally, the optical fiber (606) can be used as sensor. For example, a pulse of light may be delivered to the cochlear tissues through the optical fiber (606). In response, the tissues may reflect a portion of the light pulse or generate an electrical signal in response to the light pulse. The reflected light can be captured by the optical fiber (606). This light can be processed during the insertion process to provide active feedback. For example, the timing, intensity, and/or wavelength of the reflected light may indicate the distance between the tip of the electrode array and the cochlear tissues, the health of the tissues, or other parameters.

Fig. 6C shows an optical fiber (606) with a ball lens (608) at the tip of the optical fiber (606). The ball lens (608) collimates the light (610) emitted from the optical fiber (606). This and other fiber optic designs could be used for a variety of purposes, including providing nerve stimulation and/or use as an endoscope. The optical fibers may be temporarily connected to an exterior controller that is not part of the cochlear implant. The external controller could inject light energy into the cochlea through the optical fiber and/or receive light energy out of the cochlea for analysis or display. In other examples, the optical fibers may be connected to and controlled by the implanted processor. In some examples, the optical fiber comprises infrared optical fiber that is transparent at wavelengths greater than 1.5 microns. This allows the passage of light frequencies that directly simulate nerve tissue.

As discussed above, the cochlea is configured to detect high frequency tones near the base and progressively lower frequencies near the apex. In some implementations, the tip electrodes could be used after the insertion is complete to provide additional low frequency content to the cochlea, either as an analog signal or an amplitude modulated pulse string.

In general, a cochlear electrode array with tip elements includes a flexible body and an array of electrodes disposed along a longitudinal surface of the flexible body. A tip element is disposed at a distal end of the flexible body. The tip element lies on a distal surface of the flexible body and is configured to stimulate nerves proximate to the distal end of the flexible body during insertion of the cochlear electrode array into the cochlea. The tip element may include one or more of: at least one conductive electrode (such as a platinum tip electrode), a split electrode, a ball electrode, an optical fiber with a first end terminating at the distal end of the flexible body, and combinations thereof. Where the tip element includes an optical fiber, the optical fiber may be an infrared optical fiber that is transparent at wavelengths greater than 1.5 microns. The tip element may also include various optical components such as a fixed or steerable lens, such as shown in Fig. 6D, described below.

Tip elements could be included in a variety of cochlear electrodes. For example, the tip elements could be included in a precurved electrode, such as modiolar hugging electrodes, or in electrodes that are substantially straight, such electrode that are designed for lateral placement within the cochlea.

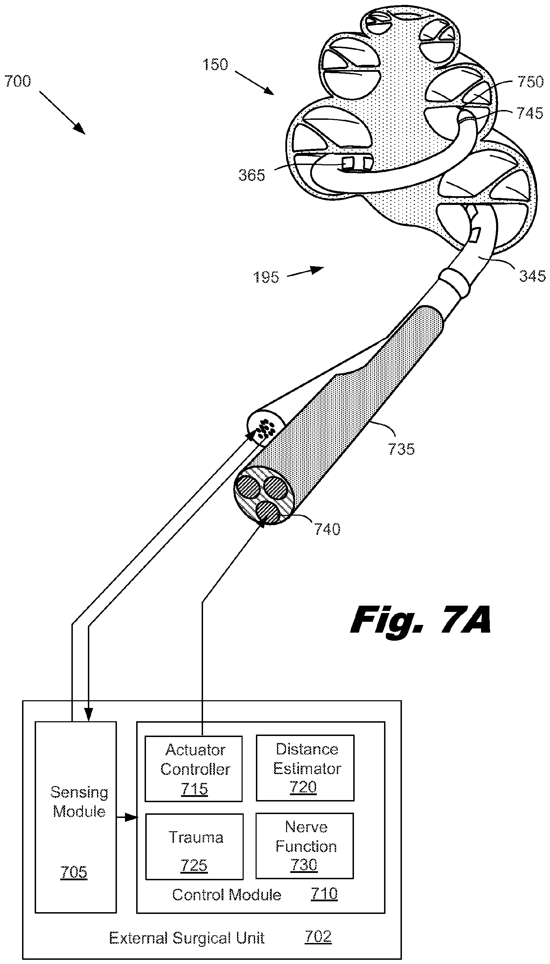

Fig. 7A is a diagram of a system (700) for detecting the surroundings of a cochlear electrode during insertion and using the data to improve patient outcomes. Automated cochlear implant insertion techniques and methods are described in U.S. Pat. App. Pub. No. 20110114288 entitled "Cochlear Electrode insertion" to Matthew I. Haller et al., which is incorporated herein by reference in its entirety.

The illustrative system shown in Fig. 7A includes a cochlear electrode array (195), an insertion tool (735) and an external surgical unit (702). As discussed above the cochlear electrode array (195) may include flexible body (345), a plurality of medial electrodes (365) disposed along one side of the flexible body, and tip elements (745, 750). In this example, the tip elements include a ring electrode (745) and an optical fiber (750) that terminates at the distal end of the flexible body.

Only the distal portion of the insertion tool (735) that engages with the electrode array (195) is shown in this diagram. The insertion tool (735) may be any of a variety of tools, including standard manual surgical implements, specialty manual surgical tools that are specifically adapted for insertion of a cochlear implant, or surgical tools that are at least partially automated. Examples of surgical tools that are at least partially automated include surgical tools that are held and manipulated by the surgeon's hand but provide haptic feedback and/or limited amount of automated motion. For example, actuators in the surgical tool may compensate for a hand shake, provide motion that is not easily performed by hand, or automatically advance the electrode into the cochlea. In other examples, the actuator may be fully automatic. Actuators can provide a number of advantages, including sensitivity, accuracy, speed and control that are outside of the capabilities of the human hand. In this example, a cross section of the insertion tool (735) shows three actuators (740).

In this implementation, the external surgical unit (702) includes a sensing module (705) and a control module (710). The sensing module (705) is connected to tip elements (745, 750). Additionally, the sensing module (705) may be connected to the linear array of electrodes (365). The sensing module (705) can provide stimulus to the tip elements. For example, the sensing module (705) may provide optical energy of desired wavelengths, intensities, and duration to the optical fiber (750). The sensing module (705) may also supply electrical voltage/current to the tip electrode (745). For example, the sensing module (705) may stimulate nerves by sending a pulse of infrared light down the optical fiber. The optical fiber transmits the light to the target nerve tissue. The nerve tissue is stimulated to produce an action potential which is detected by the tip electrode (745). One or more of the linear electrodes (365) can be used as a ground for this measurement. The sensing module (705) senses the voltage produced by the stimulated nerves.

The sensing module (705) transmits its results to the control module (710). The control module (710) may include a number of sub units. For example, the control module (710) may include an actuator controller (715), a distance estimator (720), a trauma unit (725) and a nerve function unit (730). The actuator controller (715) can be used to provide: input to the mechanical actuators (740), haptic output, audio output, graphical output or any other suitable action. The description of actuators given in Fig. 7A is just one example. As discussed above, the insertion tool may be entirely manual. In other examples, the insertion tool and/or electrode array may include at least one actuator. In the implementation shown in Fig. 6D, the optical fiber terminates at a steered optic (612). The actuator controller (715) could be used to steer the lens to direct the light (614) to the desired location or angle (615).

The distance estimator (720) can output any of a number of products based on the input from the sensing module. For example, the distance between the tip of the electrode array and the wall of the cochlea can be measured in a variety of ways. In one example, light pulses are sent down the optical fiber. The light pulses exit the optical fiber and a portion of the light pulses reflect back into the optical fiber. These reflections are detected by the sensor module and the distance module calculates the distance between the tip of the electrode and the cochlear wall. Additionally or alternatively, the distance and/or contact of the electrode array with the cochlea can be measured using electrical capacitance, audio techniques or other suitable methods. This distance information can be used by the control module to provide the desired output/control.

The trauma unit (725) can be used to detect changes in the cochlea that indicate that trauma has occurred during the insertion of the electrode array. For example, the trauma unit may accept the nerve performance data from the sensor module and determine if changes in the function of the cochlea are related to trauma. In other examples, trauma may be detected directly. For example, tissue that has been exposed to trauma may react differently to incident light or electrical stimulation. The control module may use this information to control the actuators to mitigate the trauma.

The nerve function unit (730) may produce a map of the function of the cochlea as the electrode array is inserted. This map may be used for a variety of purposes, including detecting trauma resulting from surgery and determining the best way to use the electrodes to improve the hearing of the patient. For example, if the map shows that the patient has significant residual hearing in a particular range of frequencies, the insertion of the electrode array can be modified to minimize trauma in this region. Additionally, the map may be used as an input during programming of the implanted processor. For example, after the electrode array is in place and the cochlear implant is functional, the processor can be programmed to provide supportive stimulation in frequency ranges where there is still residual hearing and to provide replacement stimulation where residual hearing is minimal.

The sensing nature of the design can also be used to monitor the long term health of regions of the cochlea and the auditory nerve. These measurements could be used to drive (re)programming of the cochlear implant as the function of certain cochlear regions or the auditory nerve itself changes.

Fig. 7A is an illustrative example of a system that includes an electrode array with tip elements. The principles described herein could be implemented in a variety of ways, including systems that have more, less, or different functionality. For example, if the insertion does not include actuators, the actuator controller may be omitted. Additionally, the modules and units are presented to illustrate one potential implementation. The functions of the modules and units could be combined in different ways. In general, the external surgical unit can be implemented using a computer processor, memory, communication buses, and other electronic components. In some implementations, the external surgical unit may include specialized electronics that make up the sensing module and actuator controller.

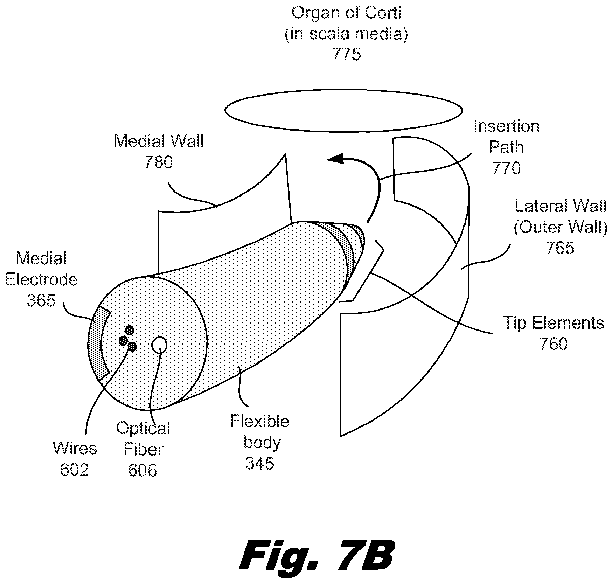

In each example described above, the tip elements are in a different plane than the linear array of medial electrodes and are configured to stimulate or sense different portions of the cochlea. Fig. 7B shows a perspective view of a flexible body (345) with medial electrodes (365) disposed along one side and tip elements (760) located on the most distal portion of the flexible body. The cross section of the flexible body shows the individual wires (602) that are connected to the electrodes and an optical fiber (606).

The flexible body (345) is inserted through the scalae timpani along an insertion path (770). As the flexible body (345) is inserted into the ascending spiral of the scalae timpani, the flexible body bends so that the medial electrodes (365) are oriented toward the medial wall (780) along the length of the flexible body. Specifically, the medial electrodes (602) are generally parallel to the medial wall (780) and are the closest part of the flexible body to the medial wall. As discussed above, this places the array of medial electrodes (365) proximal to the spiral ganglion in the modiolus.

In contrast, the tip elements (760) are located at the most distal end of the flexible body (345) and are not solely configured to stimulate the spiral ganglion. Specifically, the tip elements (760) are oriented in a plane that is different than the plane of the array of medial electrodes and are configured to stimulate (or sense) nerve activity in directions other than the medial direction. The tip elements may be oriented to stimulate or sense: the area in front of the electrode array; the organ of Corti (775) (typically 90 degrees up or down from the medial direction); or the outer wall of the cochlea (180 degrees away from the medial wall).

When the tip elements (760) are oriented to sense the area in front of the electrode array they may produce information about any structural blockages that might be in the cochlear channel or the auditory response of the cochlear nerves prior to contact by the electrode array. When the tip elements (760) are oriented to stimulate/sense response by the organ of Corti (775) in locations adjacent to the tip of the electrode array, the tip simulators (760) may be sensing the immediate impact of the electrode array insertion on the function of the cochlea. When negative responses are detected during insertion, the electrode array can be backed out prior to causing irreversible trauma. A different insertion approach can then be taken to advance the electrode array into the cochlea. When the tip elements (760) are configured to sense the outer wall (785) of the cochlea, the tip elements can be used to measure the distance between the tip element and the wall or other parameters. This can be useful in atraumatic insertion of the electrode array and provide feedback to guide electrode array away from the lateral cochlea wall (765).

The tip elements (760) can be differentiated from the electrodes in a variety of ways. For example, the electrodes (365) are in a linear array when the flexible body are straight and are adjacent to and pointed directly toward the medial wall (780) when the flexible body is inserted into the cochlea. Thus, during and after insertion of the flexible body into the cochlea, a normal vector representing the orientation of the medial electrodes would intersect the modiolus. In contrast, the tip elements will typically be oriented around the circumference of the tip or on a forward face of the tip of the flexible body. A normal vector representing the orientation of tip elements will not typically intersect the modiolus. For example, a normal vector representing the orientation of a given element could be calculated by dividing the exposed surface of the element into sections, determining a normal vector for each section, and then summing the normal vectors to produce a resulting vector.

Further, the electrodes in the array are configured to stimulate the spiral ganglion. In contrast, the tip elements are configured to stimulate/sense areas that are in front of the flexible body, above the flexible body (as shown in Fig. 7B, where the organ of Corti in the current spiral is above the flexible body), below the flexible body (where the organ of Corti for the previous spiral is located), or to sense the lateral wall (785). Thus, the electrodes are configured to concentrate/direct electrical stimulus toward the spiral ganglion while the tip elements are configured to direct stimulating energy in other directions and toward different structures. For example, the non-uniform distribution of electromagnetic energy by the split electrodes (400) shown in Fig. 5B preferentially simulates locations above and below the electrode array. Consequently, the tip elements can be distinguished from electrodes by their location, orientation, and function.

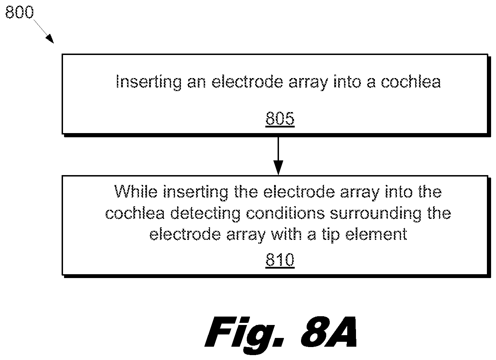

Fig. 8A is a flow chart of an illustrative method (800) for using an electrode array with a tip element. The electrode array is inserted into a cochlea (block 805) through a cochleostomy or through an opening in the round window. While inserting the electrode array into the cochlea, conditions surrounding the electrode array are detected using the tip element (block 810.) In some examples, the tip element may be used to detect conditions that are in front of the electrode array. This can be helpful in detecting obstacles or conditions that could be encountered as the electrode array continues to advance into the cochlea. In other examples, the tip element(s) could be used to detect the conditions that surround the distal tip of the electrode array.

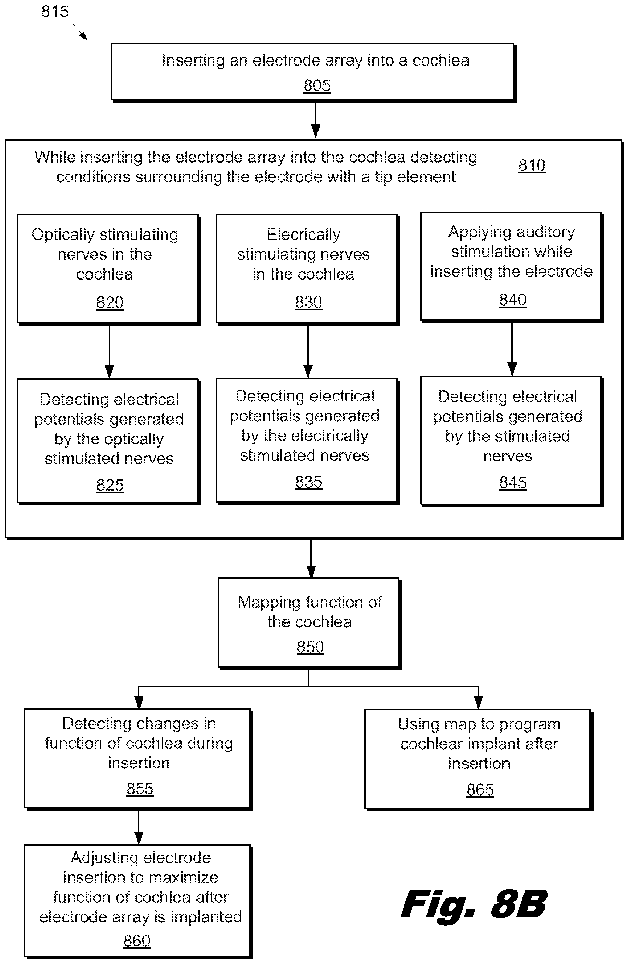

Fig. 8B is a more detailed flowchart of a method (815) for using an electrode array with a tip element. As discussed above, the electrode array is inserted into the cochlea (block 805) and, while inserting the electrode array into the cochlea, the conditions surrounding the electrode array are detected with a tip element (block 810). The tip element(s) can be any of a number of devices, including devices that emit or sense light, sound, electricity, or other energy. In some examples, the tip element may be entirely passive, such as microphone or electrode that senses energy produced by the cochlea. In other examples, the tip elements may be either active elements or they may be used as both detectors and elements. For example, an optical fiber may be used as an active element that simulates nerve tissue, a detector of light, or both. In some examples, the optical fiber may be used as an endoscope to view the interior of the cochlea. This can provide valuable visual input to the surgeon during surgery.

In one example, an optical fiber is used to stimulate nerves in the cochlea (block 820) and then electrical potentials generated by the optically stimulated nerves can be detected by an electrode (block 825). In another example, nerves in the cochlea can be stimulated using a electrode tip element (block 830) and then the electrical potentials generated by the stimulated nerves can be detected by the same or a different tip electrode (block 835).

In yet another example, auditory stimulation may be applied while inserting the electrode (block 840). The auditory stimulation may be produced externally to the ear or internally by a transducer. The transducer may be temporarily placed on or near the cochlea during surgery or may be an integral part of the cochlear electrode. The transducer may produce a wide variety of sound waves with varying pitches and intensities. As the nerves in the cochlea respond to the auditory stimulation, the action potentials produced can be sensed using the tip electrodes.

The descriptions above are related to sensing the function of the cochlea. A variety of other measurements can also be made. For example, the fiber optic may be used to directly or indirectly view portions of the cochlea that are in front of the tip electrode.

After mapping the function of the cochlea (block 850), changes in the function can be detected during insertion (block 855). In some examples, reversible trauma can be detected and the electrode insertion adjusted to maximize the function of the cochlea after the electrode array is implanted (block 860). Additionally or alternatively, the map of the cochlear function can be used to program the cochlear implant after insertion (block 865).

The methods, techniques and systems described above are only examples. Principles incorporated in these methods and systems could be applied in a variety of ways. For example, an electrode array that includes an imaging fiber optic may be implanted in a patient and subsequently require revision surgery to remove the electrode array. The imaging fiber optic may be used to during the revision surgery to image the interior of the cochlea as the electrode array is withdrawn. The imaging information may include images of structures and obstructions within the cochlea. Alterations in the structure of cochlea may have occurred during the period the electrode array was implanted. For example, there may be some tissue growth or alteration of the cochlea in response to the presence of the electrode array, aging of the patient, ongoing disease, or other factors. The imaging of these alterations can provide a number of benefits including better selection and programming of replacement cochlear implant or other diagnostic purposes. Similarly, if the tip element includes electrodes, the electrodes may be used to produce a map of the function of the cochlea as the electrode array is withdrawn. This map may be compared to a map that was produced during insertion to evaluate changes in the function of the cochlea.

In sum, incorporating a tip element or elements into a cochlear electrode array can provide for stimulation and detection of the surroundings of the cochlear electrode array during insertion. This allows for the function of the cochlea to be mapped and adjustments made during the insertion of the array. This can preserve residual hearing of the patient and optimize the results of the cochlear implant.

The preceding description has been presented only to illustrate and describe examples of the principles described. This description is not intended to be exhaustive or to limit these principles to any precise form disclosed. Many modifications and variations are possible in light of the above teaching. The features shown and/or described in connection with one figure may be combined with features shown and/or described in connection with other figures.

## Claims

1. A cochlear implant system comprising:
an electrode array (195) and a sensing module (705),
the electrode array comprising a tip element (400, 745, 750, 760), the tip element being a device that emits or senses light, sound or electricity; and
the sensing module being configured to be in communication with the tip element, wherein the sensing module is configured to detect conditions surrounding the electrode array using the tip element during insertion of the electrode array into a cochlea,
**characterized in that** the sensing module (705) comprises a nerve function unit (730) configured to map cochlear nerve function proximate to the tip of the electrode array (195) during insertion of the electrode array into a cochlea.

2. The system of claim 1, further comprising a control module (710) to receive output from the sensing module (705) and provide feedback during insertion of the electrode array (195) into the cochlea.

3. The system of claim 1, further comprising actuators (740) to manipulate the electrode array (195) during insertion into the cochlea, wherein the actuators are controlled by the control module (710) based on the output of the sensing module (705).

4. The system of claim 1, wherein the tip element comprises an optical fiber (606) with a first end and a second end, wherein the first end is connected to the sensing module (705) during insertion of the electrode array (195) into the cochlea and the second end (750) terminates at a distal end of the electrode array.

5. The system of claim 1, wherein the tip element comprises an electrode (400, 745) or an optical fiber (606, 750).

6. The system of claim 1, wherein the sensing module comprises a distance estimating unit (720) configured to measure a distance to a cochlear structure during insertion of the electrode array (195) into a cochlea.

7. The system of claim 1, wherein the sensing module (705) comprises a trauma unit (725) configured to measure reversible trauma to the cochlea during insertion of the electrode array.

8. The system of claim 1, wherein the sensing module (705) is configured to detect conditions surrounding the electrode array (195) by
stimulating auditory nerves with the tip element (400, 745, 750) while moving the electrode array by electrical stimulation or by optical stimulation with wavelengths of light greater than 1.5µm (1.5 microns); and
detecting, with the tip element, the auditory potentials generated by the nerves while inserting the electrode array.

9. The system of claim 8, wherein the tip element comprises an optical fiber (606, 750) configured to transmit wavelengths of light greater than 1.5 microns and an electrode (745) configured to detect auditory potentials produced by optical stimulation of nerves.

10. The system of claim 8, wherein the tip element comprises at least one conductive electrode (400, 745), wherein stimulating nerves in the cochlea comprises placing a voltage on the at least one conductive electrode such that auditory potentials are generated in a nerve proximate the conductive electrode.

11. The system of claim 1, wherein the tip element comprises an optical fiber (606, 750) and wherein the system is configured to detect conditions surrounding the electrode array by sending a light pulse through the optical fiber, the light pulse exiting the optical fiber and striking the interior of the cochlea.

12. The system of claim 8, wherein the system is configured to detect conditions surrounding the electrode array (195) by detecting reversible decline in auditory response caused by mechanical contact between the electrode array and structures in the cochlea.

13. The system of claim 1, wherein the system is configured to activate the tip element (400, 745, 750, 760) to stimulate auditory nerves when the electrode array is stationary within the cochlea.

14. The system of claim 1, further comprising: a flexible body (345); wherein the electrode array is an array of medial electrodes (365) disposed along one side of the flexible body, the array of medial electrodes lying in a surface substantially parallel to a medial wall of a cochlea; wherein the tip element (400, 745, 750, 760) is disposed at a distal end of the flexible body, and wherein the tip element does not lie in the same surface as the medial electrodes and is configured to stimulate nerves proximate the distal end of the flexible body during insertion of the cochlear electrode array into the cochlea.

## Patentansprüche

1. Cochlea-Implantat mit:
einer Elektrodenanordnung (195) und einem Sensormodul (705),
wobei die Elektrodenanordnung ein Spitzenelement (400, 745, 750, 760) aufweist, bei welchem es sich um eine Vorrichtung handelt, die Licht, Schall oder Elektrizität emittiert oder erfasst; und
wobei das Sensormodul ausgebildet ist, um mit dem Spitzenelement zu kommunizieren und um Bedingungen um die Elektrodenanordnung herum während des Einführens der Elektrodenanordnung in eine Cochlea unter Verwendung des Spitzenelements zu erfassen,
**dadurch gekennzeichnet, dass** das Sensormodul (705) eine Nervenfunktionseinheit (730) aufweist, die ausgebildet ist, um eine Cochlea-Nervenfunktion nahe der Spitze der Elektrodenanordnung (195) während des Einführens der Elektrodenanordnung in eine Cochlea abzubilden.

2. System gemäß Anspruch 1, ferner versehen mit einem Steuermodul (710) zum Empfangen eines Ausgangssignals von dem Sensormodul (705) und zum Liefern einer Rückmeldung während des Einführens der Elektrodenanordnung (195) in die Cochlea.

3. System gemäß Anspruch 1, ferner versehen mit Aktuatoren (740) zum Manipulieren der Elektrodenanordnung (195) während des Einführens in die Cochlea, wobei die Aktuatoren von dem Steuermodul (710) basierend auf dem Ausgangssignal des Sensormoduls (705) gesteuert werden.

4. System gemäß Anspruch 3, wobei das Spitzenelement eine optische Faser (606) mit einem ersten Ende und einem zweiten Ende aufweist, wobei das erste Ende während des Einführens der Elektrodenanordnung (195) in die Cochlea mit dem Sensormodul (705) verbunden ist und das zweite Ende (750) an einem distalen Ende der Elektrodenanordnung endet.

5. System gemäß Anspruch 1, wobei das Spitzenelement eine Elektrode (400, 745) oder eine optische Faser (606, 750) aufweist.

6. System gemäß Anspruch 1, wobei das Sensormodul eine Abstandsabschätzeinheit (720) zum Messen eines Abstands zu einer Cochlea-Struktur während des Einführens der Elektrodenanordnung (195) in eine Cochlea aufweist.

7. System gemäß Anspruch 1, wobei das Sensormodul eine Traumaeinheit (725) zum Messen von reversiblem Trauma der Cochlea während des Einführens der Elektrodenanordnung aufweist.

8. System gemäß Anspruch 1, wobei das Sensormodul (705) ausgebildet ist, um Bedingungen um die Elektrodenanordnung (195) herum zu erfassen, indem
Hörnerven mit dem Spitzenelement (400, 745, 750) mittels elektrischer Stimulation oder optischer Stimulation mit Lichtwellenlängen von mehr als 1,5 µm (1,5 Mikrons) stimuliert werden, während sich die Elektrodenanordnung bewegt; und
mit dem Spitzenelement die Hörpotentiale erfasst werden, die von den Nerven während des Einführens der Cochlea-Anordnung erzeugt werden.

9. System gemäß Anspruch 8, wobei das Spitzenelement eine optische Faser (606, 750), die zum Abstrahlen von Lichtwellenlängen von mehr als 1,5 Mikrons ausgebildet ist, sowie eine Elektrode (745) aufweist, die ausgebildet ist, um Hörpotentiale zu erfassen, die mittels optischer Stimulation von Nerven erzeugt werden.

10. System gemäß Anspruch 8, wobei das Spitzenelement mindestens eine leitende Elektrode (400, 745) aufweist, wobei beim Stimulieren von Nerven in der Cochlea eine Spannung an der mindestens einen leitenden Elektrode so angelegt wird, dass Hörpotentiale in einem Nerv nahe der leitenden Elektrode erzeugt werden.

11. System gemäß Anspruch 1, wobei das Spitzenelement eine optische Faser (606, 750) aufweist und wobei das System ausgebildet ist, Bedingungen um die Elektrode herum zu erfassen, indem ein Lichtpuls durch die optische Faser geschickt wird, welcher aus der optischen Faser austritt und das Innere der Cochlea trifft.

12. System gemäß Anspruch 8, wobei das System ausgebildet ist, um Bedingungen um die Elektrodenanordnung (195) herum zu erfassen, indem eine reversible Abnahme der Hörantwort erfasst wird, die durch mechanischen Kontakt zwischen der Elektrodenanordnung und Strukturen in der Cochlea verursacht wird.

13. System gemäß Anspruch 1, wobei das System ausgebildet ist, um das Spitzenelement (400, 745, 750, 760) zu aktivieren, um Hörnerven zu stimulieren, wenn die Elektrodenanordnung innerhalb der Cochlea stationär ist.

14. System gemäß Anspruch 1, ferner versehen mit einem flexiblen Körper (345), wobei es sich bei der Elektrodenanordnung um eine Anordnung von medialen Elektroden (365) handelt, die entlang einer Seite des flexiblen Körpers angeordnet sind, wobei die Anordnung von medialen Elektroden in einer Oberfläche im Wesentlichen parallel zu der medialen Wand einer Cochlea liegt, wobei das Spitzenelement (400, 745, 750, 760) an einem distalen Ende des flexiblen Körpers angeordnet ist, und wobei das Spitzenelement nicht in der gleichen Oberfläche wie die medialen Elektroden liegt und ausgebildet ist, um Nerven nahe des distalen Endes des flexiblen Körpers während des Einführens der Cochlea Elektrodenanordnung in die Cochlea zu stimulieren.

## Revendications

1. Système d'implant cochléaire comprenant :
un réseau d'électrodes (195) et un module de détection (705),
l'ensemble d'électrodes comprenant un élément de pointe (400, 745, 750, 760), l'élément de pointe étant un dispositif qui émet ou détecte de la lumière, un son ou de l'électricité ; et
le module de détection étant configuré pour être en communication avec l'élément de pointe, dans lequel le module de détection est configuré pour détecter des conditions entourant l'ensemble d'électrodes à l'aide de l'élément de pointe pendant l'insertion de l'ensemble d'électrodes dans une cochlée,
**caractérisé en ce que** le module de détection (705) comprend une unité de fonction nerveuse (730) configurée pour mapper la fonction nerveuse cochléaire à proximité de la pointe de l'ensemble d'électrodes (195) pendant l'insertion de l'ensemble d'électrodes dans une cochlée.

2. Système selon la revendication 1, comprenant en outre un module de commande (710) destiné à recevoir une sortie en provenance du module de détection (705) et à fournir une rétroaction pendant l'insertion de l'ensemble d'électrodes (195) dans la cochlée.

3. Système selon la revendication 1, comprenant en outre des actionneurs (740) destinés à manipuler l'ensemble d'électrodes (195) pendant l'insertion dans la cochlée, dans lequel les actionneurs sont commandés par le module de commande (710) en se basant sur la sortie du module de détection (705).

4. Système selon la revendication 1, dans lequel l'élément de pointe comprend une fibre optique (606) ayant une première extrémité et une seconde extrémité, dans lequel la première extrémité est raccordée au module de détection (705) pendant l'insertion de l'ensemble d'électrodes (195) dans la cochlée et la seconde extrémité (750) se termine au niveau d'une extrémité distale de l'ensemble d'électrodes.

5. Système selon la revendication 1, dans lequel l'élément de pointe comprend une électrode (400, 745) ou une fibre optique (606, 750).

6. Système selon la revendication 1, dans lequel le module de détection comprend une unité d'estimation de distance (720) configurée pour mesurer une distance par rapport à une structure cochléaire pendant l'insertion de l'ensemble d'électrodes (195) dans une cochlée.

7. Système selon la revendication 1, dans lequel le module de détection (705) comprend une unité de traumatisme (725) configurée pour mesurer un traumatisme réversible à la cochlée pendant l'insertion de l'ensemble d'électrodes.

8. Système selon la revendication 1, dans lequel le module de détection (705) est configuré pour détecter des conditions entourant l'ensemble d'électrodes (195)
en stimulant des nerfs auditifs avec l'élément de pointe (400, 745, 750) tout en déplaçant l'ensemble d'électrodes au moyen d'une stimulation électrique ou d'une stimulation optique ayant des longueurs d'onde de lumière supérieures à 1,5 µm (1,5 microns) ; et
en détectant, avec l'élément de pointe, les potentiels auditifs générés par les nerfs tout en insérant l'ensemble d'électrodes.

9. Système selon la revendication 8, dans lequel l'élément de pointe comprend une fibre optique (606, 750) configurée pour transmettre des longueurs d'onde de lumière supérieure à 1,5 microns et une électrode (745) configurée pour détecter des potentiels auditifs produits par une stimulation optique de nerfs.

10. Système selon la revendication 8, dans lequel l'élément de pointe comprend au moins une électrode conductrice (400, 745), dans lequel la stimulation de nerfs dans la cochlée consiste à placer une tension sur la ou les électrodes conductrices de telle sorte des potentiels auditifs soient générés dans un nerf à proximité de l'électrode conductrice.

11. Système selon la revendication 1, dans lequel l'élément de pointe comprend une fibre optique (606, 750) et dans lequel le système est configuré pour détecter des conditions entourant l'ensemble d'électrodes en envoyant une impulsion lumineuse à travers la fibre optique, l'impulsion lumineuse sortant de la fibre optique et atteignant l'intérieur de la cochlée.

12. Système selon la revendication 8, dans lequel le système est configuré pour détecter des conditions entourant l'ensemble d'électrodes (195) en détectant un déclin réversible d'une réponse auditive provoqué par un contact mécanique entre l'ensemble d'électrodes et des structures dans la cochlée.

13. Système selon la revendication 1, dans lequel le système est configuré pour activer l'élément de pointe (400, 745, 750, 760) pour stimuler des nerfs auditifs lorsque l'ensemble d'électrodes est fixe à l'intérieur de la cochlée.

14. Système selon la revendication 1, comprenant en outre : un corps flexible (345) ; dans lequel l'ensemble d'électrodes est un ensemble d'électrodes médianes (365) disposées le long d'un côté du corps flexible, l'ensemble d'électrodes médianes se trouvant sur une surface sensiblement parallèle à une paroi médiane d'une cochlée ; dans lequel l'élément de pointe (400, 745, 750, 760) est disposé au niveau d'une extrémité distale du corps flexible et dans lequel l'élément de pointe ne se trouve pas sur la même surface que celle des électrodes médianes et est configuré pour stimuler des nerfs à proximité de l'extrémité distale du corps flexible pendant l'insertion de l'ensemble d'électrodes cochléaires dans la cochlée.
